# EUROPEAN PATENT APPLICATION

(11) **EP 4 029 525 A1**
(43) Date of publication of application: **20.07.2022**
(21) Application number: 21151308.0
(22) Date of filing: 13.01.2021
(51) Int. Cl.: A61L 2/00, A61L 2/10, A61L 2/24, E03D 9/00, E05B 1/00, A47K 13/00

(54) **USE OF AT LEAST ONE COLOR RADIATION SOURCE IN A SYSTEM COMPRISING A RADIATION BODY**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); NIESSEN, Eduard Matheus Johannes, 5656 AE Eindhoven (NL); Martens, Peter, 5656 AE Eindhoven (NL)
(74) Representative: Philips Intellectual Property & Standards

(57) **Abstract**

A system (1) that comprises a radiation body (10) comprising an exit radiation window (11) to be disinfected under the influence of UV radiation (23) is equipped with a radiation arrangement (20) and a controller arrangement (40). The radiation arrangement (20) comprises at least one UV radiation source (21, 22) configured to emit UV radiation (23) and at least one color radiation source (24, 25) configured to emit visible light (26) of at least one color, and the controller arrangement (40) serves to control operation of both the at least one UV radiation source (21, 22) and the at least one color radiation source (24, 25). When the system (1) is also equipped with a detector arrangement (30), it is possible to detect touch of a relatively large object (5) on the radiation exit window (11) and to reduce intensity of the UV radiation (23) to a safe level.

## Description

### FIELD OF THE INVENTION

The invention relates to a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body; and a radiation arrangement configured to provide the radiation.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element, an optical sensor and a control system. The waveguide element comprises a radiation exit window, wherein the waveguide element is i) configured to receive radiation, wherein the radiation at least comprises UV radiation, ii) configured to radiate at least part of the radiation to the exterior of the waveguide element via the radiation exit window, and iii) configured to internally reflect part of the radiation at the radiation exit window. The optical sensor is configured to sense an internal reflection intensity of the internally reflected radiation. The control system is functionally coupled to the optical sensor and is configured to reduce the intensity of the radiation as function of reaching a predetermined first threshold of a reduction of the internal reflection intensity over time.

WO 2018/215272 A1 teaches that the UV radiation is used for killing microorganisms as may be present on the radiation exit window, or for rendering the microorganisms inactive or unable to reproduce. Practical examples of microorganisms are bacteria. The disinfecting effect of using UV radiation is mainly governed by a total dose of the UV radiation. In the context of using UV radiation, it may be necessary to take specific measures when higher organisms, including human beings, may be in a position of receiving the UV radiation, which is especially the case if it is possible for the higher organisms to physically contact radiation emitting surfaces.

During operation of the known system, radiation is coupled out of the waveguide element through the radiation exit window if some object touches the window. This outcoupling means that less radiation will stay inside the waveguide element and is also referred to as frustration of the (total) internal reflection. Therefore, by using the optical sensor to monitor an internal reflection intensity of the internally reflected radiation, it is possible to detect a situation of some object touching the window, especially some object that is considerably larger than microorganisms, such as a human hand or finger. When a considerable step is observed in the signal provided by the optical sensor, it is assumed that this means that a relatively large object contacts the window. In order to ensure safety in such a situation, it may be determined that the radiation needs to be shut off, at least temporarily.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications of the invention that is the subject of WO 2018/215272 A1, it is important that external surfaces are kept in a disinfected state while situations in which the UV radiation used in the process might cause harm to higher organisms such as human beings need to be avoided.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. However, the same principle of outcoupling the radiation at contact positions would cause potentially unsafe exposure of human beings touching the waveguide element to UV radiation if it was not for the measure of detecting the touching and shutting off the radiation. In the context of the present invention, it is acknowledged that there is a need for developing useful ways of actually putting this measure to practice. Also, in view of the fact that UV radiation is not visible to the human eye, it is acknowledged that there is a need for visually alerting human beings to the use of UV radiation in the system.

### SUMMARY OF THE INVENTION

It is an object of the invention to design a system comprising a radiation body and a radiation arrangement in such a way that a proper balance is realized between effectively obtaining disinfection results at the position of a radiation exit window of the radiation body on the one hand and protecting human beings and other higher organisms from harmful exposure to UV radiation provided by at least one UV radiation source of the radiation arrangement on the other hand. Also, it is an object of the invention to design a system comprising a radiation body and a radiation arrangement in such a way that it is possible to reliably alert a human being to the use of UV radiation in the system. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation and visible light of at least one color, and wherein the radiation exit window is capable of allowing at least part of the radiation to pass to the exterior of the radiation body; a radiation arrangement configured to provide the radiation, comprising at least one UV radiation source configured to emit UV radiation and at least one color radiation source configured to emit visible light of at least one color; and a controller arrangement, functionally coupled to the radiation arrangement to control operation of both the at least one UV radiation source and the at least one color radiation source.

In a first aspect, the invention covers an option of such a system comprising a detector arrangement configured to detect intensity of at least one of the UV radiation and the visible light of the at least one color at at least one detector position in the radiation body, wherein the controller arrangement is functionally coupled to the detector arrangement and configured to control operation of at least the at least one UV radiation source in dependence on input including input received from the detector arrangement. In this respect, it may particularly be so that the detector arrangement is configured to detect intensity of at least the visible light of the at least one color at the at least one detector position, and further that the controller arrangement is configured to set a normal state of the at least one UV radiation source in which the at least one UV radiation source provides the UV radiation with an intensity at an operational level as a default, and to put the at least one UV radiation source from the default state to an adapted state in which the at least one UV radiation source provides the UV radiation with an intensity at a level that is reduced relative to the operational level after occurrence of a disturbance incident involving a change of the intensity of at least the visible light of the at least one color detected by the detector arrangement at the at least one detector position. The reduced level of the intensity of the radiation provided by the radiation source in the adapted state may be a zero level, although this is not necessary in the context of the invention.

In the context of the first aspect of the invention, the safety measure as mentioned involves detection and assessment of behavior of visible light of at least one color. In the context of the invention, the term "visible light of at least one color" is to be understood so as to refer to visible light of a certain wavelength or range of wavelengths. As is generally known, the color of visible light varies with the wavelength of the light. Further, it is noted that the indication that the detector arrangement is configured to detect intensity of radiation/light at at least one detector position may imply in practice that the detector arrangement is capable of obtaining data for calculating and outputting an arbitrary parameter related to the respective intensity at the at least one detector position, wherein it is not necessary that the detector arrangement is capable of determining the actual value of the internal radiation intensity at the at least one detector position.

Occurrence of a disturbance incident is determined when a change of the intensity of at least the visible light of the at least one color detected by the detector arrangement at the at least one detector position is found. The invention covers various options which may be at the basis of a change of the intensity as mentioned and which are related to the constitution of the system, particularly the features of the radiation body, including the option of such a change resulting from an increase of light exiting the radiation body, the option of such a change resulting from an increase of light being reflected into the radiation body, and the option of such a change resulting from a combination of a change of exiting light and a change of incoming light.

The controller arrangement of the system according to the invention is configured to set a normal state of the at least one UV radiation source as a default. Further, the controller arrangement is configured to process input including input received from the detector arrangement and possibly also including input received from one or more other components of the system such as an internal clock in such a way that occurrence of a disturbance incident can be determined. This allows the controller arrangement to set an adapted state of the at least one UV radiation source. In this way, assuming that occurrence of a disturbance incident is indicative of the radiation exit window being contacted by a relatively large object such as a human hand or finger, the controller arrangement is configured to enable disinfection of the radiation exit window as a default, and to implement the safety measure of reducing the intensity at which UV radiation is provided whenever appropriate. As a result, the general disinfection functionality of the system is only limited to a minimum extent while safety can be guaranteed at all times. In this respect, it is noted that the system may be designed in any appropriate way to realize/enable restoration of the default state of the at least one UV radiation source in any possible suitable fashion after the adapted state of the at least one UV radiation source has been set, and that the controller arrangement may particularly be configured to maintain the adapted state of the at least one UV radiation source only during a limited amount of time.

In a practical embodiment of the system according to the invention, the controller arrangement is configured to i) determine at least one safety test value that is representative of a parameter of the intensity of at least the visible light of the at least one color detected by the detector arrangement at the at least one detector position, ii) assess whether the at least one safety test value is inside or outside of a safety range of values, and iii) determine occurrence of a disturbance incident when the at least one safety test value shifts from inside the safety reference range of values to outside of the safety reference range of values. According to a first practical option, the at least one safety test value may be representative of a value of the intensity of at least the visible light of the at least one color detected by the detector arrangement at the at least one detector position. According to a second practical option, the at least one safety test value may be representative of a derivate of the value of the intensity of at least the visible light of the at least one color detected by the detector arrangement at the at least one detector position, particularly a value of change of the intensity in predetermined direction (i.e. to a higher value or a lower value). In respect of the first practical option, the controller arrangement may be configured to assess whether or not a value representing the value of the intensity is above or below an applicable threshold. In respect of the second practical option, the controller arrangement may be configured to calculate a value representing a difference between values of the intensity of subsequent detection actions and to assess whether or not the value is larger than an applicable threshold and/or to calculate a value representing a rate of change of the intensity in predetermined direction and to assess whether or not the value is larger than an applicable threshold.

In the context of the first aspect of the invention, it may be so that the radiation arrangement comprises at least one group of color radiation sources including at least a first color radiation source configured to emit visible light of a first color and a second color radiation source configured to emit visible light of a second color that is distinctly different from the first color. In such a case, it is advantageous if the controller arrangement is configured to i) determine at least one safety test value in respect of each of at least the visible light of the first color and the visible light of the second color, ii) determine at least one ratio of the respective safety test values, iii) assess whether the at least one ratio is inside or outside of a safety range of rations, and iv) determine occurrence of a disturbance incident when the at least one ratio shifts from inside the safety reference range of ratios to outside of the safety reference range of ratios. By performing the intensity detection in respect of different colors of the visible light and assessing how the safety test values relating to the respective colors relate to each other, it is possible to determine occurrence of a disturbance incident with high accuracy, taking into account color aspects of an object as may be present on the radiation exit window.

In fact, color aspects of an object can already be taken into account when only one color radiation source is used. For example, when one red radiation source configured to emit visible light of red color is used, it may be expected that much more of the red light is reflected when an object with light skin color is present on the radiation exit window than when an object of, for example, a green color is present on the radiation exit window. The references relied upon by the controller arrangement can be chosen such that occurrence of a disturbance incident is determined only in the first case and not in the second case. When at least two different color radiation sources are used, the color discrimination aspect in determining occurrence of a disturbance incident can be applied with higher accuracy in view of the fact that more information is available. For example, when a red radiation source configured to emit visible light of red color and a blue radiation source configured to emit visible light of blue color are used, effects on both the reflection of the red light and the blue light from the presence of an object on the radiation exit window are at the basis of determining occurrence of a disturbance incident. The information about the blue light may help to have a further clearly defined color window in respect of the object as may be in a position on the radiation exit window, and to exclude white objects, for example, when it comes to determining occurrence of a disturbance incident. In any case, by performing detection on the basis of colored light, it is possible to further maximize the disinfection functionality without compromising safety, as it is possible to rule out situations which do involve contact of a relatively large object to the radiation exit window but do not involve contact of bare skin to the radiation exit window. Thus, when the invention is put to practice in this way, it may be seen that the intensity of the UV light is only reduced in instances of contact of bare skin to the radiation exit window and not when contact to the radiation exit window is made through clothing, for example.

In the case that at least two different color radiation sources are used, it is not necessary for the detector arrangement to be equipped with different detectors, one for each color. Instead, it is possible to have a so-called broadband detector and obtain detection results per color, namely by powering the respective color radiation sources one or after another, wherein the time that each of the color radiation sources is powered does not need to be long, and may be in the order of 1 millisecond, for example. In general terms of the controlling functionality of the controller arrangement, it may be so that the controller arrangement is configured to control the group of color radiation sources to successively realize different color radiation situations involving an on state of only one of the respective color radiation sources of the group and an off state of the remainder of the respective color radiation sources of the group, and to control the detector arrangement to detect the intensity of the visible light of the respective color in each of the different color radiation situations.

In general, when the procedure of detecting occurrence of a disturbance incident relies on the use of visible light in the system, as suggested in the foregoing, it is not necessary that the at least one color radiation source that is involved in the detection is switched on all the time. Instead, it is very well possible to perform the detection during short operation periods of the at least one color radiation source, at appropriate intervals, wherein the operation periods may be as short as 1 millisecond, for example. In this way, both reliable monitoring of a touch status of the radiation exit window and practical non-visibility of the colored light can be realized.

In a second aspect, the invention covers an option of using visible light in the system to convey information to human beings in the vicinity of the system and to thereby influence their behavior towards the system, which information may particularly be related to an operation status of the at least one UV radiation source. For example, it may be practical if the radiation arrangement comprises at least one green radiation source configured to emit visible light of green color, and if the controller arrangement is configured to switch the at least one green radiation source on when the at least one UV radiation source is switched off and to switch the at least one green radiation source off when the at least one UV radiation source is switched on in such a case. In view of the fact that the color green is generally associated with safe situations, creating a generally green appearance of the radiation body by means of the at least one green radiation source only when the UV radiation source is off may help a person in the vicinity of the system to assess whether or not it may be safe to touch the radiation exit window. Similarly, it may be practical if the radiation arrangement comprises at least one red radiation source configured to emit visible light of red color, and wherein the controller arrangement is configured to switch the at least one red radiation source on and off along with the at least one UV radiation source. In view of the fact that the color red is generally associated with danger, creating a generally red appearance of the radiation body by means of the at least one red radiation source when the UV radiation source is on may help a person in the vicinity of the system to assess whether or not it may be safe to touch the radiation exit window. The system may comprise either the at least one green radiation source or the at least one red radiation source, but it may be preferred if the system comprises both the at least one green radiation source and the at least one red radiation source.

The preceding explanation of how a green radiation source and/or a red radiation source can be used in the system only refers to a few of the many practical available options. Another notable feasible option is an option of using at least one green radiation source in the system and continuously having the at least one green radiation source in an on state during operation of the system, in order to indicate to the user that the system is safe, which may especially be applicable if the system is configured to automatically reduce the intensity of the emitted UV radiation (probably to zero) when the radiation exit window is touched, as explained in the foregoing, wherein it may be advantageous to use the green visible light emitted by the at least one green radiation source for the purpose of the touch detection. Another notable feasible option relates to using at least one red radiation source and involves putting the at least one red radiation source to an on state when deviations from the default situation are detected in the system for a time longer than an applicable maximum. In the case of such deviations being caused by a person touching the radiation exit window, this may help in motivating the person to retract the respective body part from the radiation exit window. Also, at least one red radiation source may be used for indicating malfunctioning/failure of the system, indicating an empty battery or a nearly-empty battery, etc.

The second aspect of the invention is particularly relevant in cases in which the UV radiation is only used during certain disinfection periods and shut off outside of those periods, and in which it is actually possible for persons to refrain from touching the radiation exit window during at least the disinfection periods. In general, the second aspect involves a set-up of the system in which the controller arrangement is configured to set an on/off status of the at least one color radiation source in dependence on an on/off status of the at least one UV radiation source, or a high intensity/low intensity status of the at least one UV radiation source.

The number of UV radiation sources and the number of color radiation sources can be chosen freely. Likewise, in cases that the system is equipped with a detector arrangement, the number of detectors included in the detector arrangement can be chosen freely. Depending on the constitution of the radiation body and the size of the radiation exit window, it may be advantageous if the system comprises at least two UV radiation sources arranged in the radiation body at a distance from each other, if the system comprises at least two color radiation sources or groups of color radiation sources arranged in the radiation body at a distance from each other and/or if the system comprises at least two detectors arranged in the radiation body at a distance from each other. It may be practical if the radiation body comprises a slab of material, in which case the at least two UV radiation sources, the at least two color radiation sources or groups of color radiation sources and/or the at least two detectors may be embedded in the slab of material. The material of the slab of material may be silicone, for example. The at least one UV radiation source may be an LED, particularly a UV-C LED, for example. The at least one color radiation source may also be an LED.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

The above-described and other aspects of the invention will be apparent from and elucidated with reference to the following detailed description of practical embodiments of a system that is covered by the following definition: a system comprising i) a radiation body comprising a radiation exit window, wherein the radiation body is configured to receive radiation that at least comprises UV radiation and visible light of at least one color, and wherein the radiation exit window is capable of allowing at least part of the radiation to pass to the exterior of the radiation body, ii) a radiation arrangement configured to provide the radiation, comprising at least one UV radiation source configured to emit UV radiation and at least one color radiation source configured to emit visible light of at least one color, and iii) a controller arrangement functionally coupled to the radiation arrangement to control operation of both the at least one UV radiation source and the at least one color radiation source.

### BRIEF DESCRIPTION OF THE DRAWINGS

The invention will now be explained in greater detail with reference to the figures, in which equal or similar parts are indicated by the same reference signs, and in which:
Fig. 1 illustrates a first embodiment of a system according to the invention,
Fig. 2 illustrates a second embodiment of a system according to the invention, and
Fig. 3 diagrammatically shows an object that is suitable to be equipped with one or more systems of the first embodiment.

### DETAILED DESCRIPTION OF THE EMBODIMENTS

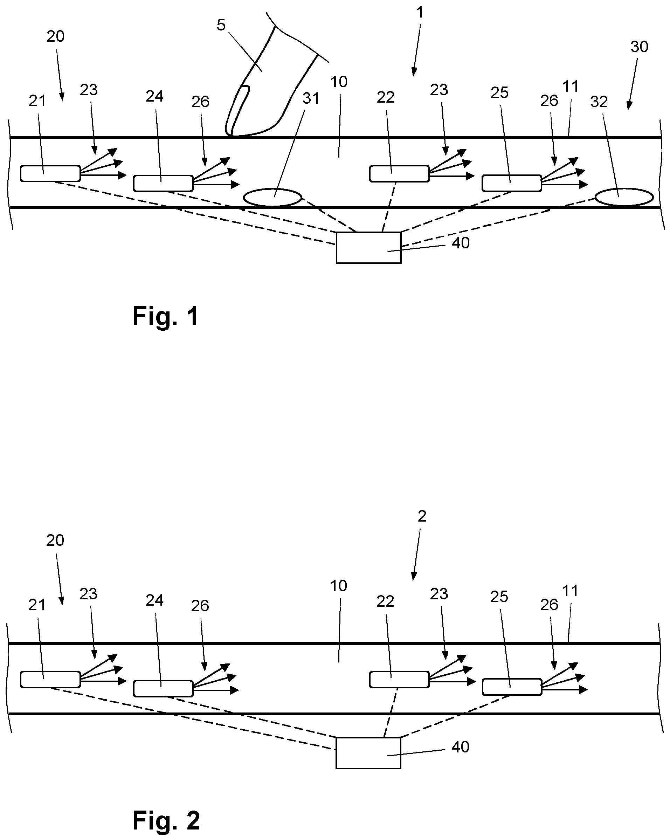

Fig. 1 illustrates a first embodiment of a system 1 according to the invention.

The system 1 comprises a radiation body 10, a radiation arrangement 20, a detector arrangement 30 and a controller arrangement 40.

The radiation body 10 comprises a radiation exit window 11. Fig. 1 illustrates the practical options of the radiation body 10 comprising a slab of material and the radiation exit window 11 being of generally flat appearance.

The radiation arrangement 20 is configured to emit radiation of at least two types, namely UV radiation such as UV-C radiation and visible light of at least one color. In the shown example, the radiation arrangement 20 comprises two UV radiation sources 21, 22 configured to emit UV radiation, in one or more directions, at least towards an interior side of the radiation exit window 11, which UV radiation sources 21, 22 may be UV-C LEDs, for example. In Fig. 1, the UV radiation emitted by the UV radiation sources 21, 22 is depicted by means of a number of arrows 23. Further, Fig. 1 illustrates the practical option of the UV radiation sources 21, 22 being embedded in the slab of material of the radiation body 10, wherein the slab of material is transparent to the UV radiation 23 provided by the UV radiation sources 21, 22. Any appropriate number of UV radiation sources 21, 22 can be chosen in the context of the invention, wherein it is possible to have an arrangement of the UV radiation sources 21, 22 in which the UV radiation sources 21, 22 are positioned to radiate the UV radiation 23 to respective areas of the radiation exit window 11 of the radiation body 10, whether or not with overlap. The UV radiation sources 21, 22 can be arranged in any appropriate pattern, including a pattern in which the UV radiation sources 21, 22 are equally distributed throughout the slab of material of the radiation body 10, in an imaginary plane extending parallel to the radiation exit window 11, in a row or in a 2D array.

Further, in the shown example, the radiation arrangement 20 comprises two color radiation sources 24, 25 configured to emit visible light of at least one color, in one or more directions, at least towards an interior side of the radiation exit window 11, which color radiation sources 24, 25 may be LEDs, for example. In Fig. 1, the visible light emitted by the color radiation sources 24, 25 is depicted by means of a number of arrows 26. Further, Fig. 1 illustrates the practical option of the color radiation sources 24, 25 being embedded in the slab of material of the radiation body 10, wherein the slab of material is transparent to the visible light 26 provided by the color radiation sources 24, 25. Any appropriate number of color radiation sources 24, 25 can be chosen in the context of the invention, wherein it is possible to have an arrangement of the color radiation sources 24, 25 in which the color radiation sources 24, 25 are positioned to radiate the visible light 26 to respective areas of the radiation exit window 11 of the radiation body 10, whether or not with overlap. The color radiation sources 24, 25 can be arranged in any appropriate pattern, including a pattern in which the color radiation sources 24, 25 are equally distributed throughout the slab of material of the radiation body 10, in an imaginary plane extending parallel to the radiation exit window 11, in a row or in a 2D array.

The detector arrangement 30 is configured to detect intensity of the visible light 26 of the at least one color. Fig. 1 illustrates the practical option of the detector arrangement 30 comprising two detectors 31, 32 which are embedded in the slab of material of the radiation body 10, whereby two detector positions are defined. Any appropriate number of detectors 31, 32 and any number of detector positions can be chosen in the context of the invention, wherein the first number nor the latter number needs to be the same as the number of color radiation sources 24, 25. The detectors 31, 32 can be arranged in any appropriate pattern, including a pattern in which the detectors 31, 32 are equally distributed throughout the slab of material of the radiation body 10, in an imaginary plane extending parallel to the radiation exit window 11, in a row or in a 2D array. In any case, if there are at least two detector positions in the radiation body 10, it is practical if the detector positions are at a distance from each other. Also, it is possible to have more than one detector 31, 32 at one detector position, or at nearly the same detector position, wherein it may be practical if the detectors 31, 32 are configured and arranged to detect light from different/opposite directions.

The controller arrangement 40 is functionally coupled to the radiation arrangement 20 and the detector arrangement 30 and is configured to control operation of the system 1. Data communication between the controller arrangement 40 and each of the UV radiation sources 21, 22, the color radiation sources 24, 25 and the detectors 31, 32 is diagrammatically indicated through dashed lines in Fig. 1. The invention covers both an option of the system 1 comprising a communication arrangement configured to enable data communication between the controller arrangement 40 and the radiation arrangement 20 and the detector arrangement 30, respectively, to take place in a wired fashion and an option of the system 1 comprising a communication arrangement configured to enable the data communication to take place in a wireless fashion, and also covers an option of the system 1 comprising a communication arrangement configured to enable the data communication to take place partly in a wired fashion and partly in a wireless fashion. The controller arrangement 40 may comprise at least one unit that is positioned outside of the slab of material of the radiation body 10, as illustrated in Fig. 1. Additionally or alternatively, it is possible that the controller arrangement 40 comprises at least one unit that is embedded in the slab of material of the radiation body 10.

The radiation arrangement 20, the detector arrangement 30 and the controller arrangement 40 may be powered in any suitable way and may be electrically coupled to the mains or to a battery, for example.

The radiation exit window 11 of the radiation body 10 is configured to transmit part of the UV radiation 23 and the visible light 26. Each of the detectors 31, 32 is at a position of receiving visible light 26 emanating from the radiation exit window 11 inward into the slab of material of the radiation body 10, and is configured to provide an output signal to the controller arrangement 40 representing intensity of the visible light 26. Depending on the structure of the radiation exit window 11, the visible light 26 that is received by the detectors 31, 32 may be obtained through at least one of i) scattering of the visible light 26 at the position of the radiation exit window 11 and ii) reflection of the visible light 26 on the interior side of the radiation exit window 11. It may be so that scattering of the visible light 26 especially takes place when an object 5 such as a person's finger is present on an exterior side of the radiation exit window 11, as diagrammatically shown in Fig. 1. In any case, when the radiation exit window 11 is contacted by an object 5, intensity of the visible light 26 traveling from the interior side of the radiation exit window 11 towards the respective detector 31, 32 changes and may in fact increase to a considerable extent.

An important function of the radiation arrangement 20 is to keep the exterior side of the radiation exit window 11 in a disinfected state. When bacteria and/or viruses end up on the exterior side of the radiation exit window 11, the bacteria and/or viruses are killed or at least rendered inactive under the influence of UV radiation 23 exiting the radiation body 10 through the radiation exit window 11 at the position of the bacteria or viruses. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or higher animals/pets.

The controller arrangement 40 is configured to control operation of the UV radiation sources 21, 22 in dependence of the signal provided by the detector arrangement 30, especially when it comes to setting the intensity of the UV radiation 23 emitted by the UV radiation sources 21, 22. The fact is that for safety reasons, it is desired to reduce the intensity of the UV radiation 23, probably to zero, in a situation of a person or higher animal/pet touching the radiation exit window 11. In view thereof, the controller arrangement 40 is configured to set a normal state of the UV radiation sources 21, 22 in which the UV radiation sources 21, 22 provide the UV radiation 23 with an intensity at an operational level as a default, and to set an adapted state of the UV radiation sources 21, 22 in which the UV radiation sources 21, 22 provide the UV radiation 23 with an intensity at a level that is reduced relative to the operational level after occurrence of a disturbance incident involving a change of output from the detector arrangement 30. As explained earlier, this output represents the intensity of the visible light 26 that is received by the respective detectors 31, 32. In this respect, it is noted that occurrence of a disturbance incident at a detector position is determined when a safety test value that is representative of a parameter of the intensity of the visible light 26 detected by the respective detector 31, 32 shifts from inside a safety reference range of values to outside of the safety range of values.

In order to ensure proper functioning of the system 1, the references relied upon in assessing occurrence of a disturbance incident are preferably chosen such that occurrence of a disturbance incident is not determined when relatively small objects are present on the radiation exit window 11, particularly objects having dimensions of tens to hundreds micrometer or even smaller dimensions, such as tiny droplets and/or grease/dirt containing bacteria and/or viruses, but only when relatively large objects 5 are present on the radiation exit window 11. The invention provides an uncomplicated and cost-effective way to combine the disinfection goal with a detection of possible presence of a higher organism, as a result of which the time that the system 1 is operated with the UV radiation sources 21, 22 in the default state is maximized and the risk of exposure of a higher organism to the UV radiation is minimized.

An interesting alternative embodiment of the system 1 is obtained if each of the color radiation sources 24, 25 is replaced by a group of color radiation sources 24, 25. For example, it is possible to apply groups of color radiation sources 24, 25 including a red radiation source, a green radiation source and a blue radiation source. In such a case, it is advantageous if the controller arrangement 40 is configured to determine at least one safety test value in respect of each of the red visible light, the green visible light and the blue visible light and to make comparisons between the safety test values in order to determine at least one relevant ratio of the test value in respect of one color versus the test value in respect of another color. Occurrence of a disturbance incident can then be determined when the at least one ratio is found to shift from inside a safety range of ratios to outside of the safety range of ratios. The references relied upon by the controller arrangement 40 in this respect can be chosen such that occurrence of a disturbance incident is determined only when the radiation exit window 11 is touched by an object 5 of a color included in a predetermined selection of colors. Thus, by choosing those colors to be colors which are related to expected skin tones, it is possible to have a functionality of the system 1 in which the disinfecting functionality is only interrupted if the radiation exit window 11 is touched by an object 5 and if the object 5 is of a color that suggests contact to bare skin. It is to be noted that also in case visible light 26 of only one color is applied in the system 1, it is possible to make a color distinction that is inherent to the system 1, but it is expected that the accuracy of doing so is improved when at least two colors are applied.

Fig. 2 illustrates a second embodiment of a system 2 according to the invention. Compared to the system 1 illustrated in Fig. 1, the system 2 illustrated in Fig. 2, which will hereinafter be referred to as signaling system 2, is without a detector arrangement 30. Hence, the signaling system 2 is without the functionality of adjusting operation of the UV radiation sources 21, 22 in response to an object 5 touching the radiation exit window 11. Instead, the color radiation sources 24, 25 are applied to convey information about the operation status of the UV radiation sources 21, 22 to human beings in the vicinity of the system 2. For example, the color radiation sources 24, 25 may be green radiation sources, in which case the color radiation sources 24, 25 may be put to an on state if the UV radiation sources 21, 22 are switched off or emit no more than an amount of UV radiation 23 that is deemed to be safe, even in view of a possible situation of an object 5 touching the radiation exit window 11, or the color radiation sources 24, 25 may be red radiation sources, in which case the color radiation sources 24, 25 may be put to an on state if the UV radiation sources 21, 22 are switched on and emit an amount of UV radiation 23 that may be harmful.

The concepts of both the first embodiment and the second embodiment may be combined if so desired. This may particularly be done by providing an embodiment in which at least one color radiation source 24, 25 is controlled to provide an appropriate signaling functionality, and in which at least one other color radiation source 24, 25 is used to provide the light 26 that may be at the basis of output from a suitable detector arrangement 30 in such a way that the system is enabled to respond to the radiation exit window 11 being touched by a relatively large object 5, particularly to cause reduction of intensity of UV radiation 23 when that happens.

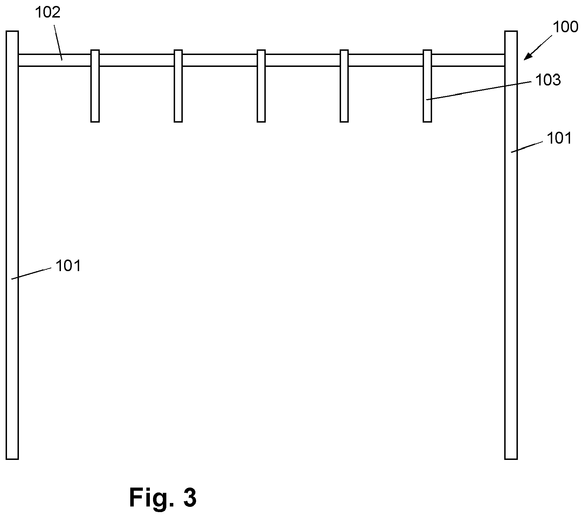

Fig. 3 diagrammatically shows an object 100 that is suitable to be equipped with one or more systems 1 of the first embodiment, as an example of the many objects included in the scope of protection of the invention. The object 100 is especially suitable to be used in a public transport vehicle such as a bus, streetcar or a train, for example, and comprises two support poles 101, a handgrip rail 102 extending between the support poles 101, and handgrip elements 103 hanging down from the handgrip rail 102. Hence, the object 100 is intended to provide support to people traveling on the public transport vehicle. People may take hold of the object 100 by gripping a support pole 101, the handgrip rail 102 or a handgrip element 103 with one or two hands and/or may lean against a support pole 101. In order to prevent spread of diseases through the object 100, it is advantageous if the systems 1 are applied to realize disinfection of the exterior of the object 100 at the position of all of the support poles 101, the handgrip rail 102 and the handgrip elements 103. Further, as the object 100 can be expected to be regularly touched by human beings, the capability of the systems 1 to automatically cause interruption of the default disinfecting functionality at the very position of touch and to thereby avoid harmful exposure of the human beings to the UV radiation 23 used for having the disinfecting functionality is most practical. The object 100 can be of conventional design, in which case the systems 1 or at least the radiation body 10 of the systems 1 and components arranged in the radiation body 10 can be arranged on an exterior surface of the object 100, or the object 100 can be of adapted design.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention are summarized as follows. A system 1, 2 that comprises a radiation body 10 comprising an exit radiation window 11 to be disinfected under the influence of UV radiation 23 is equipped with a radiation arrangement 20 and a controller arrangement 40. The radiation arrangement 20 comprises at least one UV radiation source 21, 22 configured to emit UV radiation 23 and at least one color radiation source 24, 25 configured to emit visible light 26 of at least one color, and the controller arrangement 40 serves to control operation of both the at least one UV radiation source 21, 22 and the at least one color radiation source 24, 25. When the system 1, 2 is also equipped with a detector arrangement 30, it is possible to detect touch of a relatively large object 5 on the radiation exit window 11 and to reduce intensity of the UV radiation 23 to a safe level.

## Claims

1. A system (1, 2) comprising:
- a radiation body (10) comprising a radiation exit window (11), wherein the radiation body (10) is configured to receive radiation that at least comprises UV radiation (23) and visible light (26) of at least one color, and wherein the radiation exit window (11) is capable of allowing at least part of the radiation to pass to the exterior of the radiation body (10);
- a radiation arrangement (20) configured to provide the radiation, comprising at least one UV radiation source (21, 22) configured to emit UV radiation (23) and at least one color radiation source (24, 25) configured to emit visible light (26) of at least one color; and
- a controller arrangement (40), functionally coupled to the radiation arrangement (20) to control operation of both the at least one UV radiation source (21, 22) and the at least one color radiation source (24, 25).

2. The system (1, 2) according to claim 1, comprising a detector arrangement (30) configured to detect intensity of at least one of the UV radiation (23) and the visible light (26) of the at least one color at at least one detector position in the radiation body (10), wherein the controller arrangement (40) is functionally coupled to the detector arrangement (30) and configured to control operation of at least the at least one UV radiation source (21, 22) in dependence on input including input received from the detector arrangement (30).

3. The system (1, 2) according to claim 2, wherein the detector arrangement (30) is configured to detect intensity of at least the visible light (26) of the at least one color at the at least one detector position, and wherein the controller arrangement (40) is configured to set a normal state of the at least one UV radiation source (21, 22) in which the at least one UV radiation source (21, 22) provides the UV radiation (23) with an intensity at an operational level as a default, and to put the at least one UV radiation source (21, 22) from the default state to an adapted state in which the at least one UV radiation source (21, 22) provides the UV radiation (23) with an intensity at a level that is reduced relative to the operational level after occurrence of a disturbance incident involving a change of the intensity of at least the visible light (26) of the at least one color detected by the detector arrangement (30) at the at least one detector position.

4. The system (1, 2) according to claim 3, wherein the reduced level of the intensity of the UV radiation (23) provided by the at least one UV radiation source (21, 22) in the adapted state is a zero level.

5. The system (1, 2) according to claim 3 or 4, wherein the controller arrangement (40) is configured to i) determine at least one safety test value that is representative of a parameter of the intensity of at least the visible light (26) of the at least one color detected by the detector arrangement (30) at the at least one detector position, ii) assess whether the at least one safety test value is inside or outside of a safety range of values, and iii) determine occurrence of a disturbance incident when the at least one safety test value shifts from inside the safety reference range of values to outside of the safety reference range of values.

6. The system (1, 2) according to claim 5, wherein the at least one safety test value is representative of a value of the intensity of at least the visible light (26) of the at least one color detected by the detector arrangement (30) at the at least one detector position.

7. The system (1, 2) according to claim 5 or 6, wherein the at least one safety test value is representative of a value of change in predetermined direction of the intensity of at least the visible light (26) of the at least one color detected by the detector arrangement (30) at the at least one detector position.

8. The system (1, 2) according to any of claims 5-7, wherein the radiation arrangement (20) comprises at least one group of color radiation sources (24, 25) including at least a first color radiation source (24, 25) configured to emit visible light (26) of a first color and a second color radiation source (24, 25) configured to emit visible light (26) of a second color that is distinctly different from the first color, and wherein the controller arrangement (40) is configured to i) determine at least one safety test value in respect of each of at least the visible light (26) of the first color and the visible light (26) of the second color, ii) determine at least one ratio of the respective safety test values, iii) assess whether the at least one ratio is inside or outside of a safety range of rations, and iv) determine occurrence of a disturbance incident when the at least one ratio shifts from inside the safety reference range of ratios to outside of the safety reference range of ratios.

9. The system (1, 2) according to claim 8, wherein the controller arrangement (40) is configured to control the group of color radiation sources (24, 25) to successively realize different color radiation situations involving an on state of only one of the respective color radiation sources (24, 25) of the group and an off state of the remainder of the respective color radiation sources (24, 25) of the group, and to control the detector arrangement (30) to detect the intensity of the visible light (26) of the respective color in each of the different color radiation situations.

10. The system (1, 2) according to any of claims 1-9, wherein the controller arrangement (40) is configured to set an on/off status of the at least one color radiation source (24, 25) in dependence on an on/off status of the at least one UV radiation source (21, 22).

11. The system (1, 2) according to claim 10, wherein the radiation arrangement (20) comprises at least one green radiation source (24, 25) configured to emit visible light of green color, and wherein the controller arrangement (40) is configured to switch the at least one green radiation source (24, 25) on when the at least one UV radiation source (21, 22) is switched off and to switch the at least one green radiation source (24, 25) off when the at least one UV radiation source (21, 22) is switched on.

12. The system (1) according to claim 10 or 11, wherein the radiation arrangement (20) comprises at least one red radiation source (24, 25) configured to emit visible light of red color, and wherein the controller arrangement (40) is configured to switch the at least one red radiation source (24, 25) on and off along with the at least one UV radiation source (21, 22).

13. An object (100) comprising the system (1, 2) according to any of claims 1-12, wherein the radiation exit window (11) of the radiation body (10) of the system (1, 2) is arranged to be at an exterior side of the object (100).

14. The object (100) according to claim 13, selected from a group comprising domestic appliances, furniture, construction elements of buildings and vehicles, sanitary parts, medical devices, and components of all of the foregoing.

15. Method of adding a system (1, 2) according to any of claims 1-12 to an existing object (100), wherein the radiation body (10) of the system (1, 2) is applied to an exterior surface of the existing object (100).
